# EUROPEAN PATENT APPLICATION

(11) **EP 2 826 510 A1**
(43) Date of publication of application: **21.01.2015**
(21) Application number: 13177200.6
(22) Date of filing: 19.07.2013
(51) Int. Cl.: A61M 11/06, A45D 34/02, B05B 7/00, B05B 7/24, A61L 9/14

(54) **Volatile substance diffusing device and method**

(71) Applicant: Drom Fragrances GmbH & Co. KG, 82065 Baierbrunn (DE)
(72) Inventor: White, Michael, 82065 Baierbrunn (DE); Ruizendaal, Jan-Willem, 82065 Baierbrunn (DE)
(74) Representative: Koepe & Partner

(57) **Abstract**

The present invention relates to a volatile substance diffusing device (10), comprising (a) a fluid inlet (11); (b) being in fluid communication with a nebulizer chamber (14); (c) into which a nebulizing tube (13) is extended, (d) which is in fluid communication with a volatile substance supply tank (12); (e) said nebulizer chamber (14) opening into an upper neck (15); (f) to which a silencing device (16) is fitted; (g) said silencing device (16) being in fluid communication with a hose (17); and said hose (17), at its end opposite to the silencing device (16), opens into a scent-release device (18) from which the volatile substance is released to be experienced as a mixture (19) of fluid and volatile substance by a user (20) approaching the scent-release device (18). The invention also relates to a method of diffusing a volatile substance into a fluid, said diffusion method being characterized in that (a) a volatile substance in liquid form is supplied from a container (12) to a volatile substance exit (13 a) of a nebulizing tube (13) extending into a nebulizer chamber (14), while simultaneously (b) a fluid is fed into said nebulizer chamber via a fluid inlet (11) being in fluid communication with said nebulizer chamber (14); (c) said fluid conveying said volatile substance through said nebulizer chamber (14) while mixing said volatile substance with said fluid; and (d) further conveying the volatile substance/fluid mixture through the nebulizer chamber upper neck (15) into a silencing chamber (16) for expanding and diffusing said volatile substance within said mixture; and (e) further conveying said volatile substance/fluid mixture through a hose (17) being in fluid communication with said silencing chamber (16) to a scent-release device (18), where the volatile substance diffused into the fluid is released to be experienced by a user (20) approaching the scent-release device (18). The invention also relates to the use of said device (10) for testing and other purposes.

## Description

The present invention relates to a device allowing diffusing one volatile substance or a plurality of volatile substances. In particular, the present invention relates to a volatile substance diffusing device for the public area and, more particularly, for the public area in such cases where it is desired that a number of people experience that one volatile substance or two or three or a plurality of volatile substances is/are volatilized simultaneously and brought to the persons' noses for scenting.

Moreover, the invention relates to a method of volatilizing one volatile substance or two or three or a plurality of volatile substances and bringing it/them to the nose of one person or of a plurality of persons, thereby allowing the person(s) experiencing the scent of said volatile substance(s).

Finally, the invention also relates to the use of the volatile substance diffuser device of the present invention for allowing a person or a plurality of persons experiencing that one volatile substance or two or three or a plurality of volatile substances is/are volatilized simultaneously and brought to the person's/persons' nose(s) for scenting.

In the public area, people desire to experience scenting volatile substances at numerous opportunities and on a plurality of levels:

When testing fragrances in the intention of buying a new fragrance, people often make use of an absorbent paper strip for applying a mist of fragrance of interest and allowing the solvent to evaporate, before the different fragrance notes can be scented in a concentration and mixture for evaluating the fragrance. Testing (one or more) further fragrance(s) requires a repetition of the same procedure. One disadvantage of said procedure is that a second person will always scent the same fragrance from a different absorbent paper stripe in a different concentration and composition, and identical scent experience for a plurality of persons (particularly simultaneously) will usually not be possible.

In a number of public areas (commercial show rooms, exhibition halls, spaces for assemblies or conferences, etc.), scenting flavours, fragrances and other volatile substances is provided for a number of reasons. Especially, providing opportunities of scenting flavours, fragrances and other volatile substances is nowadays often considered to be a part of a sales strategy or a part of the conference experience. For breaking such opportunities to consumers (in a commercial show room) or to attendees (in a conference centre), flavours, fragrances or other volatile substances are atomized into the air spaces, thereby filling the rooms to be experienced by every person attending. A disadvantage of such a procedure is that individually different scent experiences or scent experiences restricted only to those persons desiring them cannot be provided.

A recent change of the procedure was proposed in the industries involved: Due to the intention of providing individual scent experiences, there was a need for apparatus and devices allowing scenting flavours, fragrances and other volatile substances in public areas (commercial show rooms, exhibition halls, spaces for assemblies or conferences, etc.) which allow an individual scent experience, even a repeatable individual scent experience, of a pure volatile substance without that such flavor, fragrance or other volatile substance be sprayed to fill the whole room.

This need was satisfied with the device of the present invention allowing diffusing one or several volatile substances in order to bring it/them to a person's nose for scenting. As a result, in individual scent experience is offered, without that the whole room, space or public area is to be filled with said volatile substance. In addition, the scent experience may be repeated, upon the person's request, without that any second person in the proximity is provided with the same scent. Surprisingly, the device of the invention is also suitable for conducting tests of scenting volatile substances reliably.

Hence, the invention relates to a volatile substance diffusing device, comprising
(a) a fluid inlet;
(b) being in fluid communication with a nebulizer chamber;
(c) into which a nebulizing tube is extended,
(d) which is in fluid communication with a volatile substance supply tank;
(e) said nebulizer chamber opening into an upper neck;
(f) to which a silencing device is fitted;
(g) said silencing device being in fluid communication with a hose; and
said hose, at its end opposite to the silencing device, opens into a scent-release device from which the volatile substance is released to be experienced as a mixture of fluid and volatile substance by a user approaching the scent-release device.

Preferred embodiments of the volatile substance diffusing device are claimed in claims 2 to 9 and claims 14 and 15.

The invention also relates to a method of diffusing a volatile substance into a fluid, said diffusion method being **characterized in that**
(a) a volatile substance in liquid form is supplied from a container to a volatile substance exit of a nebulizing tube extending into a nebulizer chamber, while simultaneously
(b) a fluid is fed into said nebulizer chamber via a fluid inlet being in fluid communication with said nebulizer chamber;
(c) said fluid conveying said volatile substance through said nebulizer chamber while mixing said volatile substance with said fluid; and
(d) further conveying the volatile substance/fluid mixture through the nebulizer chamber upper neck into a silencing chamber for expanding and diffusing said volatile substance within said mixture; and
(e) further conveying said volatile substance/fluid mixture through a hose being in fluid communication with said silencing chamber to a scent-release device, where the volatile substance diffused into the fluid is released to be experienced by a user approaching the scent-release device.

Preferred embodiments of the method of the invention are claimed in claims 11, 14 and 15.

The invention also relates to the use of a volatile substance diffusing device for testing the concentration of a volatile substance or for testing the composition of components of a volatile substance or for testing the effect of a volatile substance to cover unpleasant smells or for creating a mixture of volatile substances or for experiencing a composition of visual and/or acoustic impressions together with scent impressions.

Preferred embodiments of the use are claimed in claims 13 to 15.

The present invention is now explained in detail by referring to the Figures. The Figures, and in some parts the specification as well, relate to preferred embodiments of the invention which are intended to serve for a better understanding of the invention. It is, however, understood that the invention should not be construed to be restricted to the preferred embodiments described in the specification and shown in the Figures. A skilled person will understand than many variations of the invention may be conceived which are considered to be covered by the scope of the claims attached.

In the Figures, which are not necessarily drawn true to scale,
Figure 1 shows an embodiment of the volatile substance diffusing device of the present invention;
Figure 2 shows a preferred nebulizer chamber embodiment of the volatile substance diffusing device of the present invention;
Figure 3 shows the same preferred embodiment of a nebulizer chamber as Figure 2, but particularly shows the subject-matter of claim 4;
Figure 4 shows the preferred embodiment of the silencing device of the volatile substance diffusing device of the invention;
Figures 5 A to 5 C show different exemplary shapes of the hose 17 of the volatile substance diffusing device 10; and
Figures 6 A and 6 B show exemplary shapes of the scent-release device 18 with its connected hose 17, where the scent-release device 18 has a circular shape outlet 18 a or a breathing mask-like outlet 18 b on the user's side.

The following is a detailed description of the invention and its preferred embodiments by referring to the Figures.

The terms "comprise", "comprises" or "comprising" as used in the present specification and claims, for example in claim 1 or in claim 10, has/have the meaning that the volatile substance diffusing device of the invention may comprise (i) one means or may comprise (ii) two or more means as mentioned in, for example, claim 1, or that (iii) further components, means etc. (more specifically defined below) may also be comprised by the device, i. e. by the volatile substance diffusing device as, for example, the means (e. g. a pressurizing means) claimed in claim 2 in combination with (dependent upon) claim 1. Basically the same applies to the use of the terms "comprise", "comprises" or "comprising" in the method claim, where the method may comprise one step or may comprise two or more steps as mentioned in, for example, claim 1, or that the method may comprise further steps (more specifically defined below) and, for example, derivable from the description below or from any subclaim. The same also applies to the use claims.

The terms "comprise", "comprises" or "comprising" as used in the present specification and claims may, however, also include cases where the volatile substance diffusing device of the invention mainly or even exclusively consists of those means mentioned, for example, in claim 1, optionally together with any necessary component or means a skilled person may include into such a device in order to achieve the object of the invention. Particularly in the latter case where the terms "comprise", "comprises" or "comprising" as used in the present specification and claims may have the meaning of the term "exclusively consisting of", subclaims of the present application may claim, and corresponding parts of the specification may describe, further preferred embodiments, which are characterized by additional specified features which, in combination with the features of the independent claim and corresponding parts of the description, are summarized to belong to the invention as described in its broadest scope claimed. The same applies to the method and use claims, too.

In other words: The terms "comprise" or "comprises" or "comprising" may have, in the present specification and claims, the meaning of describing a non-exhaustive enumeration of elements or features or, alternatively, may have, in the present specification and claims, the meaning of describing an exhaustive enumeration of elements, in the latter case without excluding further preferred embodiments being characterized by additional features found in the specification or claims.

Moreover, each feature described in the present specification and claimed in the claims may be combined with each other feature described, alone or in any desired combination of features, and the scope of the claims is considered to cover all such feature combinations conceivable.

The term "volatile substance", as used in the present specification and claims is understood to mean a substance which is capable of being volatized, i. e. transferred from the solid or liquid (including the dispersed and dissolved or aerosol) state into the state of a gas at conditions (preliminary conditions of temperature and pressure) at which the volatile substance diffusing device 10 is to be operated. In order to avoid that any step of varying the temperature (cooling the temperature below ambient temperature or heating the temperature above ambient temperature) or varying the pressure (reducing the pressure below ambient pressure or increasing the pressure above ambient pressure) be included into the volatilizing step conducted with the volatile substance diffusing device of the present invention, said volatizing step is assumed to be conducted at ambient temperature (i. e. a temperature of 20 ± 10 °C) and ambient pressure (i. e. a pressure of 1 bar ± 0.1 bar). Substances which can be volatized under such conditions are covered by the term "volatile substance" as used in the claims and in the present specification. Preferred embodiments of such substances are explained in detail below.

As specific examples, volatile substances include, but are not limited to fragrances, natural or nature-identical flavours, essential oils, insecticides, insect repellents, insect attractants, odor eliminators, scents covering malodours, compartment air improvers, scent improvers for linen drawers, antiseptics, deodorizers, disinfectants, air purifyers, air fresheners, aroma therapy scents, and combinations and mixtures thereof.

Moreover, the term "volatile substance" as used in the present specification and claims refers to a material or a discrete unit comprising of one or more materials that is/are vaporizable, or comprises a material that is vaporizable. The term "volatile substance(s)," thus includes, but is not limited to, compositions that are comprised entirely of a single volatile material. The terms "volatile substance(s)," "aroma," "fragrance," and "scents," as used in the present specification and claims include, but are not limited to, pleasant or savory smells, and, thus, also encompass materials that function as insecticides, insect repellants, insect attractants, air fresheners, air purifyers, disinfectants, deodorants, aromacology, aromatherapy, or any other material that acts to condition, modify, or otherwise charge the atmosphere or to modify the environment.

It should be understood that certain volatile substances and compositions including, but not limited to perfumes, aromatic materials, and scented materials, will often comprise one or more volatile substances (which may form a unique and/or discrete unit comprised of a collection of volatile materials). It should be understood that the term "volatile composition" refers to compositions that have at least one volatile component, and it is not necessary for all of the component materials of the volatile composition to be volatile. The volatile compositions described herein may, thus, also have non-volatile components. It should also be understood that when the volatile compositions are described herein as being "emitted," this refers to the volatilization of the volatile components thereof, and does not require that the non-volatile components thereof be emitted.

The term "fluid" as used in the present specification and claims is understood to mean - in the broadest sense - a substance being in the fluid state at the conditions (e. g. of temperature and pressure) and being inert, particularly chemically inert, in relation to the volatile substance or volatile substances used in accordance with the invention. In preferred embodiments of the invention, the fluid is a substance being in the gaseous state under the conditions specified above and being the usual operation conditions of the volatile substance diffusing device of the invention. But this is not restricting the term "fluid", which term may also relate to a liquid substance which is inert, particularly chemically inert, in relation to the volatile substance or volatile substances used in accordance with the invention.

The fluid has the function of dissolving or dispersing (depending on a single case) the volatile substance(s) used in the gaseous state and/or conveying said volatile substance or substances, either as a gaseous mixture or as an aerosol, wherein the fluid is the gaseous component and the volatile substance(s) is/are the liquid component(s). In preferred embodiments of the invention, the fluid is a substance in which or under which the volatile substance(s) can be stored and filled into the container 12 used in the volatile substance diffusing device 10 of the invention. Preferably, the liquid has a volatility which is higher than the volatility of the volatile substance(s) used in the present invention and does not deteriorate the process of volatilizing the volatile substance(s).

Beyond the above, a skilled person who knows such fluids and may select them freely from a suitable group of such fluids of his technical knowledge is not bound by any restrictions when selecting the fluid. Of course, the fluid may be one (chemical) substance, or the fluid may comprise two or more (chemical) substances in admixture.

In preferred embodiments of the invention, the fluid may be selected from compounds which do not have any property interfering with properties of the volatile substance(s) used in this invention, e. g. with respect to smell or flavor, disinfecting properties, insect repelling capability, malodor-covering property, property of improving compartment air, volatility etc.. Even more preferably, the fluids useable in accordance with the present invention are selected from the group consisting of air, nitrogen, oxygen, noble gases and mixtures thereof.

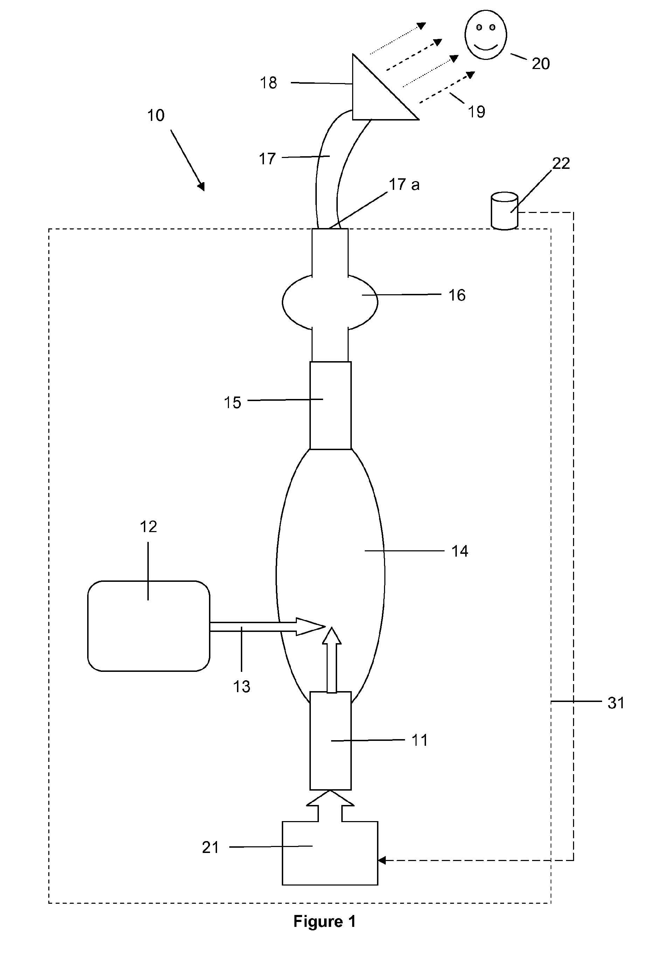

Reference is now made to Figure 1. Figure 1 shows an embodiment of the volatile substance diffusing device 10 of the present invention: The volatile substance diffusing device 10 is shown in Figure 1 in a vertical position, which is the position in which the volatile substance diffusing device is usually operated. In other words: The scent-release device 18 is positioned - usually, but not as a restriction of the invention and in any case for the present specification as a preferred embodiment - at the top of the volatile substance diffusing device 10 of the present invention.

Said volatile substance diffusing device 10 comprises as one structural component, a fluid inlet 11 which is in fluid communication with a nebulizer chamber 14 usually - but not necessarily - located above said fluid inlet 11. The fluid inlet serves to supply a fluid from a fluid supply (not shown, but well known to a skilled person so that it may be omitted in the present description). There may be one fluid supply or two or more fluid supplies, depending on the number of components of the fluid required. In a preferred embodiment of the invention, and also following the principle to provide a simple device, one fluid supply is provided in the present invention and, hence, one fluid is supplied via the fluid supply 11 into the nebulizer chamber 14.

In Figure 1, the fluid communication between the fluid inlet 11 and the nebulizer chamber is shown to be a fixed communication allowing a fluid flow between the fluid inlet 11 and the nebulizer chamber 14. It is, however, possible and is also one embodiment of the invention that the fluid inlet 11 is in fluid communication with the nebulizer chamber 14 via a tube or hose so that the fluid communication is flexible. The latter may be an advantage in cases where only a narrow space is available to accommodate the parts: A flexible relation of the parts relative to each other may then be realized. It is also possible, in another embodiment of the invention, that the connection between fluid inlet 11 and nebulizer chamber 14 is releasable, e. g. in cases of maintenance or repair. In operation, the fluid communication between the nebulizer chamber 14 and the fluid inlet 11 allows a fluid flow so that leaks etc. can be prevented.

The nebulizer chamber 14 has a size allowing a merging of fluid and volatile substance while these two components are flown in the nebulizer chamber 14. While the absolute size of the nebulizer chamber depends upon the amounts of volatile substance to be atomized/nebulized before arriving the scent-release device 18, the size of the nebulizer chamber will practically be in the range of from 10 mm to 150 mm, preferably in the range of from 10 mm to 100 mm and more preferably in the range of from 10 mm to 80 mm, seen along the vertical axis of the nebulizer chamber 14, and of from 10 mm to 70 mm and preferably of from 10 mm to 35, seen in the direction perpendicular to the vertical axis, without restricting the invention to the above proposed sizes.

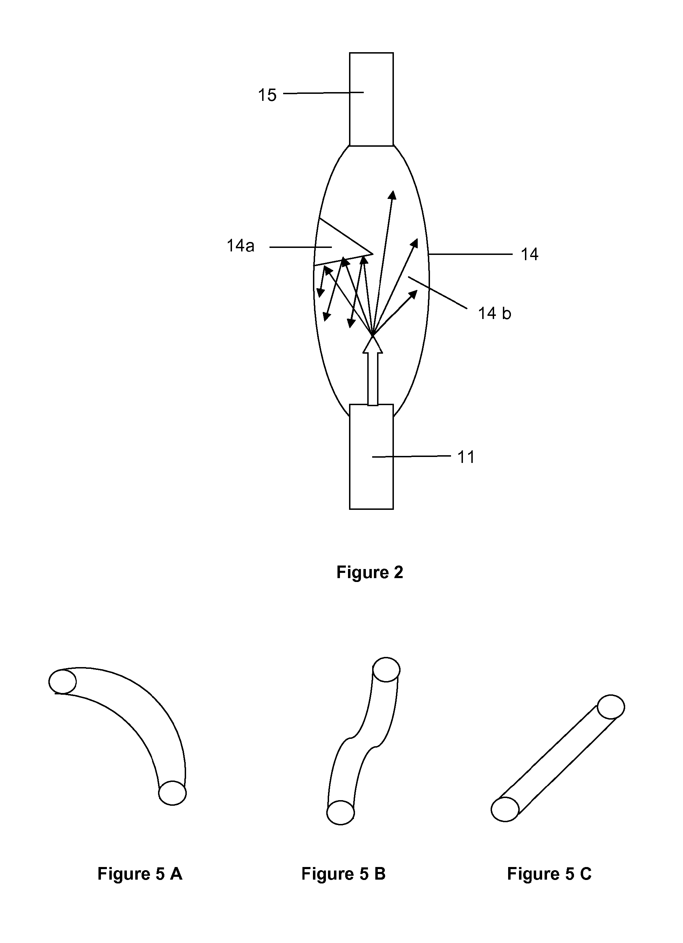

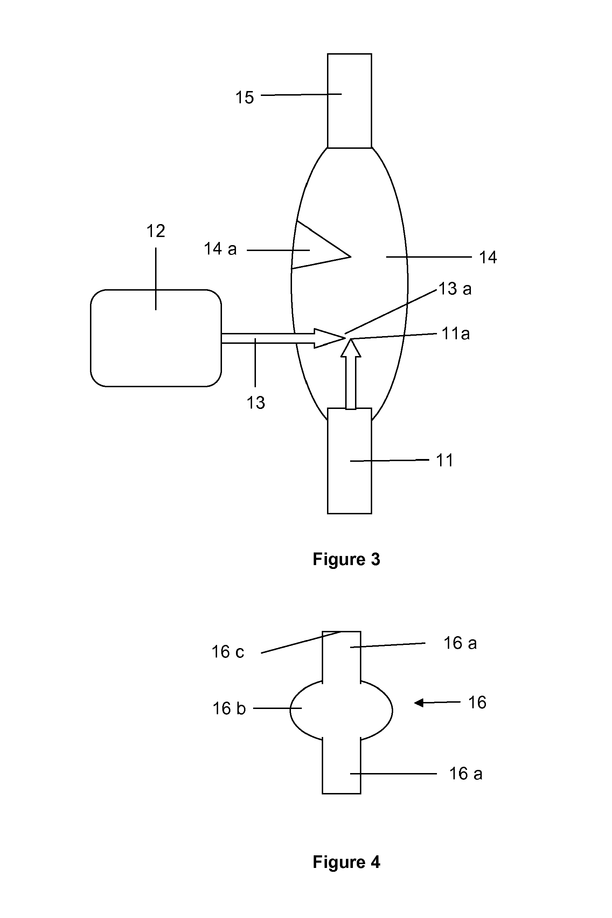

The shape of the nebulizer chamber is not restricted and, particular is not restricted to the ellipsoidal shape shown in Figure 1 (and also shown in Figures 2 and 3). A skilled person knows a large number of suitable shapes and may select such shapes in accordance of the specific requirements of a single case and in accordance with suitable shapes required under the circumstances. Suitable shapes might be a spherical shape, a ellipsoidal shape, a cylindrical shape (the latter with sharp or round corners), or any other shape conceivable. In cases of narrow spaces in which the nebulizer chamber 14 has to be accommodated, a spherical, ellipsoidal or cylindrical shape is preferable, and an ellipsoidal, spherical or cylindrical shape of the nebulizer chamber 14 is particularly preferred.

In preferred embodiments of the invention, the fluid inlet 11 is extending (at least a bit) into the inner volume of the nebulizer chamber 14. In one preferred embodiment of the volatile substance diffusing device 10 of the invention, the fluid inlet 11 is extending into the inner volume of the nebulized chamber 14 so as to be close to the nebulizing tube's 13 volatile substance exit 13 a explained immediately below.

A further structural part of the volatile substance diffusing device 10 of the present invention is a nebulizing tube 13 which is extended into the nebulizer chamber 14, as shown in Figures 1 and 3. The direction by which the nebulizing tube 13 is extended into the nebulizer chamber is not restricted and, particularly, is not restricted to the horizontal direction which is shown in Figures 1 and 3. Hence, the direction may be vertical (i. e. in parallel to the fluid inlet 11) or may be horizontal, as shown in Figures 1 and 3, or may be in any conceivable angle (to the vertical nebulizer chamber axis).

In a preferred embodiment of the invention, the nebulizing tube's 13 volatile substance exit 13a is located in close proximity to the fluid inlet orifice 11a. Such a proximity position of the nebulizing tube's 13 volatile substance exit 13 a can be realized in the way shown in Figures 1 and 3, i. e. by allowing that the tip of the volatile substance exit 13 a of the nebulizing tube 13 is close to the fluid inlet orifice 11 a which, in turn, is at the tip of the fluid inlet 11. Such a proximity position may serve allowing that the volatile substance exiting from the volatile substance exit 13a is entrained by the stream of fluid let in through the fluid inlet orifice 11 a. This will be particularly effective (and, hence, advantageous) in the case described below in detail where the fluid let in through the fluid inlet 11 and passing the fluid inlet orifice 11a is under a certain pressure and, thus, has a certain flow energy into the nebulizer chamber 14.

Such a proximity of the nebulizing tube's 13 volatile substance exit 13 a to the fluid inlet orifice 11 a may be achieved by approaching the two tips of the tubes 11, 13 in a way shown in Figures 1 and 3, i. e. at a certain angle between 45 ° (shown in the Figures) and, for example, 20 °. Another way of achieving proximity of the two tips of the tubes 11, 13 may be to bend the tip of the volatile substance exit 13 a at a certain angle; in this case, both tubes can extend into the nebulizer chamber 14 in a parallel manner, and the volatile substance exit 13 a tip is bent towards the fluid inlet orifice 11 a. By such proximity position of the two tips 11a, 13a, the fluid flow is allowed to entrain the volatile substance liquid drops supplied to the volatile substance exit 13 a of the nebulizing tube, thereby providing a good admixture and an improved diffusion of the volatile substance liquid droplets into an aerosol flowing downstream the nebulizer chamber 14.

In accordance with the invention, the nebulizing tube 13 is in fluid communication with a volatile substance supply tank 12. Said tank 12 may comprise one volatile substance or may comprise two or three or a plurality of volatile substances in admixture. As an alternative embodiment, two or three or a plurality of volatile substances may be supplied to the nebulizing tube 13 from two or three or a plurality of volatile substance supply tanks 12 (of which only one is shown in Figure 1 and in Figure 3, for reasons of simplifying the Figures). The supply may be achieved simply by the entrainment of the volatile substance(s) into the fluid flow exiting from the fluid inlet orifice 11 a. In an alternative embodiment, there may be an optional means driving the flow of volatile substance(s) towards the nebulizing tube's 13 volatile substance exit 13 a. The former embodiment is preferred due to the fact that it does not require any additional means to be installed in order to drive the volatile substance towards the nebulizing tube 13.

Also in the case of the fluid communication between the volatile substance supply tank 12 and the nebulizing tube 13, there may be a fixed connection between the tank 12 and the tube 13, allowing a volatile substance (liquid) flow between the volatile substance supply tank 12 and the nebulizing tube 13. It is, however, possible and is also one embodiment of the invention that the tank 12 is in fluid communication with the nebulizing tube 13 via a tube or hose so that the fluid communication is flexible. The latter may be an advantage in cases where only a narrow space is available to accommodate the parts: A flexible relation of the parts relative to each other may then be realized. It is also possible, in another embodiment of the invention, that the connection between volatile substance supply tank 12 and nebulizing tube 13 is releasable, e. g. in cases of maintenance or repair. In operation, the fluid communication between the nebulizing tube 13 and the volatile substance supply tank 12 allows a liquid flow so that leaks etc. can be prevented.

In the downstream direction of (in Figures 1, 2 and 3: above) the nebulizer chamber 14, said nebulizer chamber 14 opens into an upper neck 15. To the downstream end (in the Figures 1, 2 and 3: to the upper end in the vertical direction), a silencing device 16 is fitted in accordance with the invention. The silencing device 16 is fixed (preferably: releasably fixed) to the nebulizer chamber's 14 upper neck 15. Such a connection advantageously will allow a volatile substance (liquid)/fluid mixture flow between the nebulizer chamber's 14 upper neck 15 and the silencing device 16. It is, however, possible and is also one embodiment of the invention that the silencing device 16 is in fluid communication with the nebulizer chamber's 14 upper neck 15 via a tube or hose so that the fluid communication is flexible. The latter may be an advantage in cases where only a narrow space is available to accommodate the parts: A flexible relation of the parts relative to each other may then be realized. It is also possible, in another embodiment of the invention, that the connection between the nebulizer chamber's 14 upper neck 15 and the silencing device 16 is releasable, e. g. in cases of maintenance or repair. In operation, the fluid communication between the nebulizer chamber's 14 upper neck 15 and silencing device 16 allows a continuous flow of the volatile substance/fluid mixture so that leaks etc. can be prevented.

The silencing device 16 downstream the nebulizer chamber's 14 upper neck 15 has the purpose of dampening or reducing any noise caused by the flow of fluid, e. g. by the flow of air, from the fluid inlet 11, particularly if the latter, in one preferred embodiment, is provided with a pressurizing means 21 or compressor 21 as described below in detail, to the nebulizer chamber 14. Also the nebulizing process as such, described below in detail, may cause a certain noise, which can be dampened or reduced by the silencing device 16.

In a preferred embodiment of the invention which might be realized as an additional feature alone or in combination with other features of the invention and which best meets the requirements of silencing, the silencing device 16 comprises a central tube 16 a which, in an even more preferred embodiment of the invention, has approximate cylinder shape in the (vertical) flow direction, e. g. around a vertical axis, although such cylinder shape is not a restriction for the present invention: Other shapes are also conceivable by a skilled person who will select the shape of the central tube 16 a in accordance with the requirements of a specific case. In said preferred embodiment of the invention, the cylinder shape central tube 16 a widens into a spherical or ellipsoidal or cylindrical silencing space 16 b arranged coaxially with the central tube 16 a, as shown in Figures 1 and 4. Such shape preferably allows easy damping of noise coming from the nebulizer chamber 14 during the step of volatilizing the volatile substance during the nebulizing process described below.

In accordance with the invention, the silencing device 16 is in fluid communication with a hose 17. Preferably, the silencing device 16 is fixed (preferably releasably fixed) to the hose 17 by the silencing device's 16 downstream (in the Figures: upper) end. Such a connection advantageously will allow a volatile substance (liquid)/fluid mixture to flow between the silencing device 16 and the hose 17. It is, however, possible and is also one embodiment of the invention that the silencing device 16 is in fluid communication with the hose 17 via a tube or hose so that the fluid communication is flexible. The latter may be an advantage in cases where only a narrow space is available to accommodate the parts: A flexible relation of the parts relative to each other may then be realized. It is also possible, in another embodiment of the invention, that the connection between silencing device 16 and the hose 17 is releasable, e. g. in cases of maintenance or repair. In operation, the fluid communication between the silencing device 16 and the hose 17 allows a continuous flow of the volatile substance/fluid mixture so that leaks etc. can be prevented.

In accordance with the invention, said hose 17, at its end opposite to the silencing device 16, opens into a scent-release device 18, as shown in Figure 1. From said scent-release device 18, the volatile substance, in its mixture with the conveying fluid, is released to be experienced as a mixture 19 of fluid and volatile substance by a user 20 approaching the scent-release device 18.

In a preferred embodiment of the invention, which might be realized as an additional feature alone or in combination with other features of the invention and may provide advantages in relation to the step of diffusing volatile substances for the scenting experience, the volatile substance diffusing device 10 according the invention further comprises means 21 allowing pressurizing a fluid and feeding said pressurized fluid to the fluid inlet 11. A skilled person in this technical field knows such means 21 due to his/her expertise in this field and may select such a means (or a plurality of such means) in accordance with the requirements of a specific case. Hence, such means may be selected from those means generally known without restriction. In an even more preferred embodiment of the invention, said means 21 allowing pressurizing a fluid is a compressor 21, and such a compressor 21 may be provided in a way to be in fluid communication to the fluid inlet 11 and, even more preferred, supplies pressurized fluid or pressurized fluids to the fluid inlet 11.

In such a preferred embodiment of the invention, there is no need that a large overpressure be applied to the fluid/fluids or that the fluid/fluids be put under permanent overpressure. In accordance with said preferred embodiment, a slight overpressure, more preferably a slight overpressure in the range of from 1.02 bar to 2.0 bar, is applied to the fluid/fluids with the aim of allowing the fluid/fluids to entrain the volatile substance(s) emerging from the nebulizing tube 13 into a fluid(s)/volatile substance(s) mixture being further conveyed downstream in the volatile substance diffusing device 10 to be scented by the user 20 when being released from the scent-release device 18.

In another - likewise preferred - embodiment of the volatile substance diffusing device 10 of the invention, the pressurizing means/compressor 21 provides a short overpressure "push" to the fluid inlet 11 so as to send a predetermined amount of fluid or fluids through the fluid inlet 11 into the nebulizer chamber 14. This is an advantageous embodiment of the volatile substance diffusing device 10 of the invention, because said predetermined amount of fluid/fluids entrains a predetermined amount of volatile substance(s) from the nebulizing tube 13 into the nebulizer chamber 14 and, finally, to the scent-release device 18.

In another preferred embodiment of the invention which might be realized as an additional feature alone or in combination with other features of the invention, the means 21 allowing pressurizing a fluid and feeding said pressurized fluid to the fluid inlet 11 is capable of being actuated and interrupted by a switch 22. The switch 22 capable of actuating and interrupting an operation of the pressurizing means 21 is also shown in Figure 1. In a preferred embodiment of the invention, such a switch may be a usual ON/OFF switch, for example providing electrical power to the pressurizing means for its operation for a time when the switch is ON. In another preferred embodiment, the switch 22 may be a switch providing electrical power to the pressurizing means 21 for the time interval when the switch is pressed and interrupts providing electrical power to the pressurizing means 21 when released. In the latter case, fluid pushes may be generated by the pressurizing means 21 and sent via the fluid inlet 11 (and its fluid inlet orifice 11 a) into the nebulizer chamber 14 for effectively diffusing the volatile substance(s) and bringing it/them to the downstream scent-release device 18 for the user's 20 scent experience. Such means for actuating pressurizing means are known to a skilled person and may be selected in accordance with the requirements needed, without imposing any restriction to the invention.

In a further preferred embodiment of the volatile substance diffusing device 10 of the invention, which might be realized as an additional feature alone or in combination with the other features of the invention, the nebulizer chamber 14 comprises a fluid flow barrier 14 a against which the fluid/volatile substance mixture resulting from mixing the fluid emerging from the fluid inlet orifice 11 a and the volatile substance droplets emerging from the nebulizing tube's 13 volatile substance exit 13 a impinges. This embodiment is considered to be a particularly advantageous feature in view of the fact that a thorough mixing of fluid(s) and volatile substance(s) fed into the nebulizer chamber 14 via the fluid inlet 11 and its orifice 11 a and the nebulizing tube 13 and its volatile substance exit can be achieved. As can best be seen from Figure 2, the fluid flow is directed into directions exemplified by the arrows 14 b. When impinging against barrier 14 a, the fluid/volatile substance mixture flow will be reversed, resulting into turbulences which improve the mixing of fluid(s) and volatile substance(s). While creating turbulences, no substantial restriction of the flow diameter has to be expected, thereby effecting mixing without the need to enhance the pressure.

In another preferred embodiment of the volatile substance diffusing device 10 of the invention, which might be realized as an additional feature alone or in combination with the other features of the invention described above, the hose 17 extending between the silencing device 16 and the scent-release device 18 and being in fluid communication not only with the silencing device 16 but also with the scent release device 18 of the present volatile substance diffusing device 10 is bendable and preferably is self-standing and bendable. For having said property, the hose 17 may be made of any material or material combination allowing such bendability and, optionally, also self-standing property: A skilled person knows such materials for a hose and may select them for the hose 17 without restriction.

In an even more preferred embodiment of the invention, the hose 17 may be made of a polymer material selected from silicones (e. g. the commercial materials of the series of Tygon^{®} or Versilic^{®} of Saint Gobain Performance Plastics, Beaverton, U.S.A.) and fluorinated polymers (e. g. PTFE or PFA resins of the commercial Chemflor^{®} or Fluran^{®} series of Saint Gobain Performance Plastics, Beaverton, U.S.A.) and polyurethanes (e. g. the materials of the commercial Tygothane^{®} series of Saint Gobain Performance Plastics, Beaverton, U.S.A.) and rubbers (e. g. the rubbers of the commercial Norprene^{®} series of Saint Gobain Performance Plastics, Beaverton, U.S.A.) all suitable for hoses and tubes for use in connection with high performance applications may be used advantageously, without restricting the invention to those materials. As an alternative, the hose 17 may be made of an inner tube of a highly flexible material as, for example, a silicone, particularly preferred one commercial material of the series of Tygon^{®} silicone materials of Saint Gobain Performance Plastics, Beaverton, U.S.A., which material is surrounded by a stiffer material as, for example, an outer tube of metal rings flexible against each other, but creating a self-standing jacket around the inner flexible tube. The latter embodiment is particularly advantageous, because such a two-layer hose 17 allows a desired flexibility when used, while remaining self-standing after establishing a certain desired shape of the hose 17, for example, a bow shape (Figure 5 A), a "letter S" shape (Figure 5 B) or a straight line shape (Figure 5 C), without restricting the invention to any of these shapes, when positioning the scent-release device 18 so as to easily experiencing the scent of the volatile substance(s) emerging from said scent-release device 18.

In a further preferred embodiment of the volatile substance diffusing device 10 of the invention, which might be realized as an additional feature alone or in combination with the other features of the invention described above, the scent-release device 18 positioned downstream the volatile substance diffusing device 10 of the present invention in close proximity to the user 20 desiring to experience the scent of the volatile substance emerging at 19 from the scent-release device 18 is made of a transparent material which does not have any own smell and, preferably, can be cleaned or even disinfected easily. A skilled person knows such materials and may select one of these materials for the scent-release device 18 in accordance with the requirements of a specific case.

One preferred material is glass, and also an acrylic polymer may be used advantageously. The scent release device 18 made of one of these materials (or a combination thereof), in addition to the cleaning/disinfecting requirement, has the property of presenting a light and convenient appearance.

In a more preferred embodiment of the volatile substance diffusing device 10 of the invention, which might be realized as an additional feature alone or in combination with the other features of the invention described above, the scent-release device 18 is capable of fitting in shape the face of the user 20 desiring to experience the scent of the volatile substance emerging at 19 from the scent-release device 18. At the moment when the volatile substance(s) in admixture with the conveying fluid(s) is/are emerging from the user side opening of the scent-release device 18, the user's face will approach said opening for the scent experience. Hence, the flow of the volatile substance(s) fluid mixture is required to be "opened" towards the user's 20 face, without being released into a too broad angle. The fluid flow connection between the hose 17 and the scent-release device 18 is established by attaching the upstream (relatively narrow diameter) end of the scent-release device 18 to the downstream end of the hose 17 in a closely fitting relationship. The downstream end of the scent-release device 18 is wider in diameter than the upstream end thereof, thereby opening the flow of the volatile substance/fluid mixture towards the user's face, thereby fitting to the user's face. A skilled person knows shapes suitable for fitting with the above requirements and may select such shapes in accordance with the purposes to be met, without restricting the invention to particular shapes.

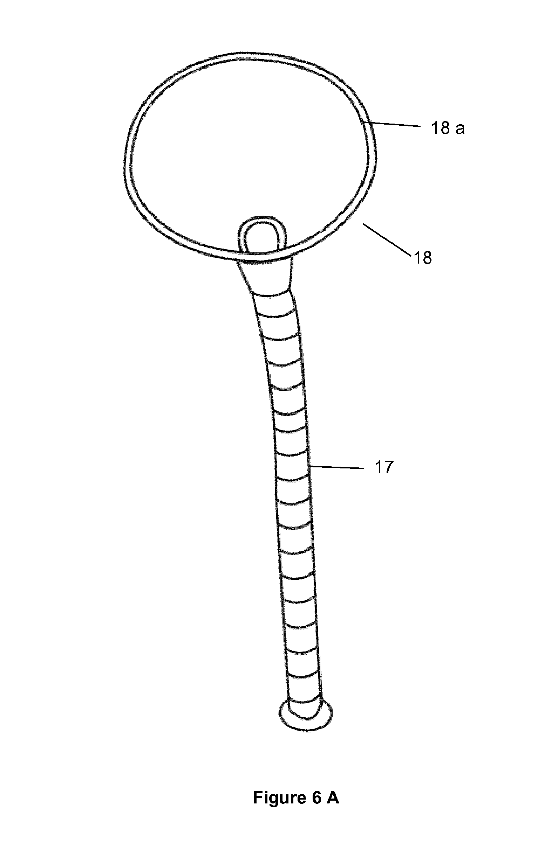

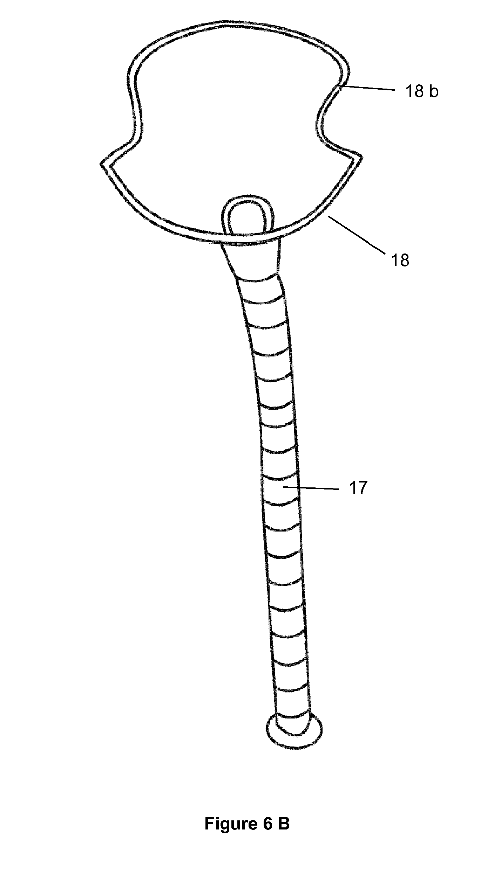

In an even more preferred embodiment of the invention, which might be realized as an additional feature alone or in combination with the other features of the invention described above, the scent-release device 18 has the shape of a funnel 18 having a circular outer opening 18 a, as shown in Figure 6 A. Alternatively (not shown in the Figures), the scent-release device 18 may have the shape of a funnel having a triangular outer opening or an opening similar to the one of a usual oxygen mask used, for example, in airplanes (see 18 b in Figure 6 B).

In another preferred embodiment of the invention, which might be realized as an additional feature alone or in combination with the other features of the invention described above, the volatile substance diffusing device's 10 components, with the exception of the hose 17 and the scent-release device 18, are housed in a housing 31, as it is shown in Figure 1 by the dotted line. In other words: Considering the volatile substance diffusing device 10 from the downstream end, the scent-release device 18 and the hose 17 are accommodated outside the housing, in said preferred embodiment, while the silencing device 16, the nebulizer chamber 14 with its upper neck 15, its nebulizing tube 13, preferably with a connected volatile substance supply tank 12 and the fluid inlet 11, optionally with its pressurizing means 21, are accommodated inside the housing 31. Of course, a skilled person may select other accommodations for the one or the other component, without that the principle of the invention is left.

This might be an advantageous embodiment in cases where the volatile substance diffusing device 10 of the invention is presented in an environment where, for presentation purposes, the technical equipment should be shielded from the user's 20 view, and only those components (e. g. the hose 17, the scent-release device 18 and the switch 22) needed for experiencing the scent of the volatile substance emerging at "19" in Figure 1 should be visible. In such a preferred case, a fluid connection 17 a between the hose 17 and the downstream end 16 c of the silencing device 16 is provided through the upper wall of the housing 31. In cases where the volatile substance diffusing device's 10 components, with the exception of the hose 17 and the scent-release device 18, are housed in a housing 31 embodied as a housing fixed to a vertical wall, the fluid connection 17 a between the hose 17 and the downstream end 16 c of the silencing device 16 may also be provided through a side wall of the housing 31.

The latter embodiment of an accommodation of the majority of components in a housing 31 may also have the switch 22 actuating the pressurizing means so as to supply the fluid into the nebulizer chamber 14 through the fluid inlet 11 mounted outside the housing 31. This embodiment can also be seen in Figure 1.

The invention also relates to a method of diffusing a volatile substance into a fluid. In accordance with the method of the invention, such a fluid may be the conveying fluid addressed already above or may be any other fluid, for example an environmental gas, as for example air, or an artificial gas in the environment, as for example nitrogen or oxygen.

The method of the invention of diffusing a volatile substance into a fluid comprises the following steps:
A volatile substance in liquid form is supplied from a container 12, wherein the volatile substance (or a plurality of volatile substances as defined below) is stored, to a volatile substance exit 13 a of a nebulizing tube 13 extending into a nebulizer chamber 14, while simultaneously
a fluid is fed into said nebulizer chamber 14 via a fluid inlet 11 being in fluid communication with said nebulizer chamber 14. Said fluid fed into the nebulizer chamber 14 has the purpose of conveying said volatile substance through said nebulizer chamber 14 while mixing said volatile substance with said fluid. Such a mixing step in accordance with the invention may be a step of dispersing a volatile substance or two or three of a plurality of volatile substances in said fluid (or in a mixture of fluids) or a step of atomizing micro-droplets of a liquid volatile substance (or of several liquid volatile substances) into said fluid in forming an aerosol of volatile substance micro-droplets in said fluid(s).

In a subsequent step, the volatile substance/fluid mixture is conveyed through the nebulizer chamber 14 upper neck 15 into a silencing chamber 16 for expanding and diffusing said volatile substance within said mixture. Subsequently, said volatile substance/fluid mixture is further conveyed through a hose 17 being in fluid communication with said silencing chamber 16 to a scent-release device 18. By means of, and through, a downstream opening of said scent-release device 18, the volatile substance(s) diffused into the fluid(s) is/are released to be experienced by a user 20 approaching the scent-release device 18 at its downstream opening, e. g. a circular opening 18 a as shown in Figure 6.

In a preferred embodiment of said method which might be realized as an additional feature alone or in combination with the other features of the invention described above, the method of the invention described above in its optional sequence of steps is conducted on a volatile substance diffusing device 10 as described above in detail. In the detailed description above and in the Figures attached, said device was described and shown in several preferred embodiments, and said description end exemplary drawings referred to above are also taken as exemplary and in detail exemplifying for the method of the invention as claimed, without that the method is restricted to these preferred embodiments.

In particular, the volatile substance diffusing device 10 of the invention may be used in a plurality of methods, where advantageous use may be made of the basic principles of the invention. The following uses are mentioned and claimed as an example for uses of the invention:
The volatile substance diffusing device 10 may be used:
   - for testing the concentration of a volatile substance in a mixture of several volatile substances or in a mixture of volatile substance(s) and one fluid or several fluids; or
   - for testing the composition of components of a volatile substance, e. g. testing in a fragrance a composition of basic notes and head notes; or
   - for testing the effect of a volatile substance to cover unpleasant smells, e. g. sweat smells resulting from bacterial decomposition of sweat; or
   - for creating a mixture of volatile substances when composing a new fragrance or an own individual fragrance; or
   - for experiencing a composition of visual and/or acoustic impressions together with scent impressions.

The above are exemplary examples are, however, not to be construed to restrict uses of the invention.

In a preferred embodiment of said use of the volatile substance diffusing device 10 which might be realized as an additional feature alone or in combination with the other features of the invention described above, the use of the invention described above in its different examples is conducted on a volatile substance diffusing device 10 as described above in detail. In the detailed description above and in the Figures attached, said device was described and shown in several preferred embodiments, and said description end exemplary drawings referred to above are also taken as exemplary and in detail exemplifying for the use of the invention as claimed, without that the use is restricted to these preferred embodiments.

In further preferred embodiments (a) of the volatile substance diffusing device as described above in detail; and (b) of the method of diffusing a volatile substance into any fluid as described above, specifically also in relation to the volatile substance diffusing device 10; and (c) of the use of diffusing a volatile substance into any fluid as described above, specifically also in relation to the volatile substance diffusing device 10, which device, method and use might be realized as an additional feature alone or in combination with the other features of the invention described above, the fluid used for diffusing, and the fluid into which the volatile substance(s) is/are diffused is selected from air, nitrogen, oxygen, noble gases and mixtures thereof.

In further preferred embodiments (a) of the volatile substance diffusing device as described above in detail; and (b) of the method of diffusing a volatile substance into any fluid as described above, specifically also in relation to the volatile substance diffusing device 10; and (c) of the use of diffusing a volatile substance into any fluid as described above, specifically also in relation to the volatile substance diffusing device 10, which device, method and use might be realized as an additional feature alone or in combination with the other features of the invention described above, the volatile substance is not restricted and preferably is one volatile substance or is two or three or a plurality of volatile substances. A skilled person in this technical field knows such volatile substances and may select them in accordance with the requirements of a specific case. Preferably the volatile substance is one, two or three volatile substance(s). In other words: Volatile substances in relation to the present invention are one isolated volatile substance or are a mixture or composition of two or three volatile substances.

In more preferred embodiments of the invention which are realized as an additional feature alone or are realized in combination with one or more of the other features of the invention described above, volatile substances diffused and treated as described above are selected from the group consisting of fragrances, natural or nature-identical flavours, essential oils, insecticides, insect repellents, insect attractants, odor eliminators, scents covering malodours, compartment air improvers, scent improvers for linen drawers, antiseptics, deodorizers, disinfectants, air purifyers, air fresheners, aroma therapy scents, and combinations and mixtures thereof. Particularly and in especial advantageous examples, preferably in connection of the uses claimed, one volatile substance is, or several volatile substances are, selected from fragrances, natural or nature-identical flavours and essential oils.

The invention was described above in detail, and preferred embodiments were also described and shown in the Figures attached. It is, however, to be emphasized that the invention is not restricted to the preferred embodiments shown in the Figures and/or described in the present specification: These preferred embodiments are exemplary for the invention and serve a better understanding thereof. The invention is not to be construed to be restricted to the preferred embodiments described. The scope of the invention is given by the claims attached.

## Claims

1. A volatile substance diffusing device (10), comprising
(a) a fluid inlet (11);
(b) being in fluid communication with a nebulizer chamber (14);
(c) into which a nebulizing tube (13) is extended,
(d) which is in fluid communication with a volatile substance supply tank (12);
(e) said nebulizer chamber (14) opening into an upper neck (15);
(f) to which a silencing device (16) is fitted;
(g) said silencing device (16) being in fluid communication with a hose (17); and
said hose (17), at its end opposite to the silencing device (16), opening into a scent-release device (18) from which the volatile substance is released to be experienced as a mixture (19) of fluid and volatile substance by a user (20) approaching the scent-release device (18).

2. The volatile substance diffusing device (10) according to claim 1, further comprising a means (21) allowing pressurizing a fluid and feeding said pressurized fluid to the fluid inlet (11), preferably wherein said means (21) allowing pressurizing a fluid is a compressor (21) being in fluid communication to the fluid inlet (11).

3. The volatile substance diffusing device (10) according to claim 2, wherein the means (21) allowing pressurizing a fluid and feeding said pressurized fluid to the fluid inlet (11) is capable of being actuated and interrupted by a switch (22).

4. The volatile substance diffusing device (10) according to any of the claims 1 to 3, wherein the nebulizing tube's (13) volatile substance exit (13a) is located in close proximity to the fluid inlet orifice (11a), thereby allowing that the volatile substance exiting from the volatile substance exit (13a) is entrained by the stream of fluid let in through the fluid inlet orifice (11 a).

5. The volatile substance diffusing device (10) according to any of the claims 1 to 4, wherein the nebulizer chamber (14) comprises a fluid flow barrier (14 a) against which the fluid/volatile substance mixture resulting from the fluid emerging from the fluid inlet orifice (11 a) and the volatile substance droplets emerging from the nebulizing tube's (13) volatile substance exit (13 a) impinges for a thorough mixing.

6. The volatile substance diffusing device (10) according to any of the claims 1 to 5, wherein the silencing device (16) comprises a central tube (16 a) which widens into a spherical or ellipsoidal silencing space (16 b) arranged coaxially with the central tube (16 a).

7. The volatile substance diffusing device (10) according to any of the claims 1 to 6, wherein the hose (17) extending between the silencing device (16) and the scent-release device (18) is bendable and preferably is self-standing and bendable.

8. The volatile substance diffusing device (10) according to any of the claims 1 to 7, wherein the scent-release device (18) is capable of fitting in shape the face of the user (20), preferably wherein the scent-release device (18) has the shape of a funnel (18) having a circular outer opening (18 a) or has the shape of a funnel having a triangular outer opening (18 b).

9. The volatile substance diffusing device (10) according to any of the claims 1 to 8, wherein the volatile substance diffusing device's (10) components, with the exception of the hose (17) and the scent-release device (18), are housed in a housing (31), and a fluid connection (17a) between the hose (17) and the downstream end (16 c) of the silencing device (16) is provided through the upper wall or the side wall of the housing (31).

10. A method of diffusing a volatile substance into a fluid, said diffusion method comprising the steps that
(a) a volatile substance in liquid form is supplied from a container (12) to a volatile substance exit (13 a) of a nebulizing tube (13) extending into a nebulizer chamber (14), while simultaneously
(b) a fluid is fed into said nebulizer chamber (14) via a fluid inlet (11) being in fluid communication with said nebulizer chamber (14);
(c) said fluid conveying said volatile substance through said nebulizer chamber (14) while mixing said volatile substance with said fluid; and
(d)further conveying the volatile substance/fluid mixture through the nebulizer chamber upper neck (15) into a silencing chamber (16) for expanding and diffusing said volatile substance within said mixture; and
(e)further conveying said volatile substance/fluid mixture through a hose (17) being in fluid communication with said silencing chamber (16) to a scent-release device (18), where the volatile substance diffused into the fluid is released to be experienced by a user (20) approaching the scent-release device (18).

11. The method of diffusing a volatile substance according to claim 10,
wherein the volatile substance diffusing device (10) according to any one of the claims 1 to 9 is used for diffusing said volatile substance(s) into the fluid.

12. Use of a volatile substance diffusing device for testing the concentration of a volatile substance or for testing the composition of components of a volatile substance or for testing the effect of a volatile substance to cover unpleasant smells or for creating a mixture of volatile substances or for experiencing a composition of visual and/or acoustic impressions together with scent impressions.

13. The use according to claim 12, wherein the diffusing device (10) according to any one of the claims 1 to 9 is used for diffusing said volatile substance(s) into a fluid.

14. The volatile substance diffusing device (10) according to any of the claims 1 to 9, or the volatile substance diffusing method according to any of the claims 10 to 11, or the use according to any of the claims 12 to 13, wherein the fluid is selected from air, nitrogen, oxygen, noble gases and mixtures thereof.

15. The volatile substance diffusing device (10) according to any of the claims 1 to 9, or the volatile substance diffusing method according to any of the claims 10 to 11, or the use according to any of the claims 12 to 13, wherein the volatile substance is one volatile substance or is two or three or a plurality of volatile substances, preferably wherein the volatile substance is one, two or three volatile substance(s), more preferably wherein the volatile substance(s) is/are selected from one or more substances selected from the group consisting of fragrances, natural or nature-identical flavours, essential oils, insecticides, insect repellents, insect attractants, odor eliminators, scents covering malodours, compartment air improvers, scent improvers for linen drawers, antiseptics, deodorizers, disinfectants, air purifyers, air fresheners, aroma therapy scents, and combinations and mixtures thereof.
